# EUROPEAN PATENT APPLICATION

(11) **EP 4 821 463 A1**
(43) Date of publication of application: **14.10.2026**
(21) Application number: 25220501.8
(22) Date of filing: 03.12.2025
(51) Int. Cl.: A61F 2/18

(54) **REPLACEMENT PROSTHESIS FOR AUDITORY OSSICLE**

(30) Priority: 08.04.2025 KR 20250045632
(71) Applicant: Kyungpook National University Industry-Academic Cooperation Foundation, Daegu 41566 (KR); Kyungpook National University Hospital, Jung-gu Daegu 41944 (KR)
(72) Inventor: LEE, Kyu-Yup, 42094 Daegu (KR); SHIN, Dongho, 41907 Daegu (KR); SEONG, Kiwoong, 42113 Daegu (KR)
(74) Representative: Mewburn Ellis LLP

(57) **Abstract**

A replacement prosthesis for an auditory ossicle, which connects an incus to a stapes, includes a head portion having one side arranged to come into contact with the stapes, and a coupler portion connected to the head portion and having one side arranged to come into contact with the incus, wherein the head portion is connected to the coupler portion such that a connection length is adjustable.

## Description

### CROSS-REFERENCE TO RELATED APPLICATION

This application is based on and claims priority under 35 U.S.C. §119 to Korean Patent Application No. 10-2025-0045632, filed on April 8, 2025, in the Korean Intellectual Property Office, the disclosure of which is incorporated by reference herein in its entirety.

### BACKGROUND

### 1. Field

The present disclosure relates to a replacement prosthesis for an auditory ossicle which is adjustable in length.

### 2. Description of the Related Art

In general, externally generated sound enters the external auditory canals and is transferred to the eardrums, and vibrations of the eardrums are transferred to inner ears through the auditory ossicles, and then converted into electrical nerve signals in the cochlea and transferred to the brain through the nerves.

In this way, the auditory ossicles play an important role in transferring sound energy while connecting the eardrums to the inner ears.

Therefore, when the auditory ossicles are damaged or their functions are not complete, a replacement prosthesis (implant) is used to replace the auditory ossicle.

However, there is a cumbersome problem that multiple replacement prostheses having various lengths have to be prepared because lengths of the damaged auditory ossicle change depending on a patient undergoing the procedure, and an appropriately sized replacement prosthesis must be selected and used during the procedure.

A related technology for the replacement prosthesis for the auditory ossicle is disclosed in Korean Patent Publication No. 10-2021-0076382 (June 24, 2021).

### SUMMARY

An objective of the present disclosure is to provide a replacement prosthesis for an auditory ossicle that is adjustable in length to fit a length that may replace the damaged auditory ossicle of a patient to be treated.

According to an aspect of the present disclosure, a replacement prosthesis for an auditory ossicle, which connects an incus to a stapes, includes a head portion having one side arranged to come into contact with the stapes, and a coupler portion connected to the head portion and having one side arranged to come into contact with the incus, wherein the head portion is connected to the coupler portion such that a connection length is adjustable.

In addition, the replacement prosthesis may further include a position fixing portion configured to fix a length-adjusted position between the head portion and the coupler portion.

In addition, the position fixing portion may use caulking to pressurize the connection portion between the head portion and the coupler portion.

In addition, the head portion may include a pipe body having a hollow, and another side of the coupler portion may be inserted into one side of the hollow.

In addition, an inner surface of the pipe body and the coupler portion may further include respectively screw portions that fasten the inner surface with the coupler portion.

In addition, the replacement prosthesis may further include an adjustment pin that is fastened with another side of the hollow by a screw portion of the adjustment pin.

In addition, the head portion may include a head body in which an insertion groove is formed, and another side of the coupler portion may be connected to the insertion groove by a screw portion of the coupler portion.

In addition, the coupler portion may include a connector shaft connected to the head portion, and a connector provided at one end of the connector shaft to come into contact with the incus.

In addition, the coupler portion may have a hook shape.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a view illustrating an installation state of a replacement prosthesis for an auditory ossicle according to an embodiment of the present disclosure;
FIG. 2 is a cross-sectional view of a replacement prosthesis for an auditory ossicle, according to an embodiment of the present disclosure; and
FIGS. 3 to 6 are cross-sectional views of a replacement prosthesis for an auditory ossicle, according to another embodiment of the present disclosure.

### DETAILED DESCRIPTION

Hereinafter, preferred embodiments according to the present disclosure will be described in detail with reference to the attached drawings. Prior to this, terms or words used in the present specification and claims should not be interpreted as being limited to their usual or dictionary meanings, and should be interpreted as meanings and concepts that are consistent with the technical spirit of the present disclosure based on the principle that an inventor may appropriately define concepts of the terms in order to explain his or her own invention in the best way.

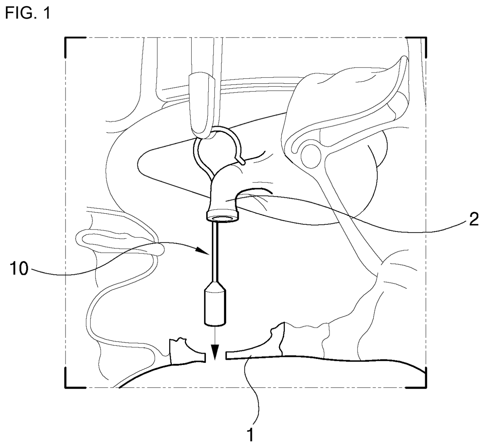

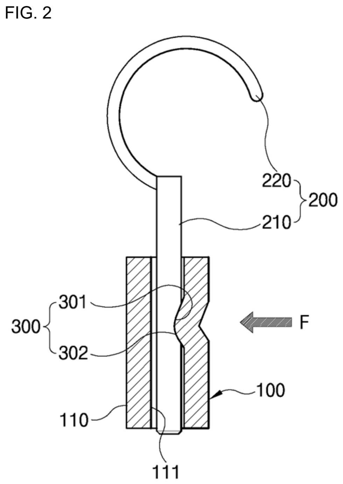

Referring to FIGS. 1 to 5, a replacement prosthesis 10 according to an embodiment of the present disclosure may include a head portion 100 and a coupler portion 200. The replacement prosthesis for an auditory ossicle, according to the embodiment is arranged to connect between a stapes 1 and an incus 2 to replace at least part of the auditory ossicle, and performs a function of stably transferring sound energy from an eardrum to an inner ear.

The head portion 100 may be arranged to be in contact with the stapes 1.

One side of the head portion 100 may be arranged to be in contact with one side of the stapes 1, and the other side of the head portion 100 may be connected to the coupler portion 200.

The head portion 100 may include a pipe body 110 having a hollow 111 that is open at both ends.

For example, the hollow 111 may be formed in a structure in which the hollow 111 is open on one side and the other side in a longitudinal direction of the pipe body 110.

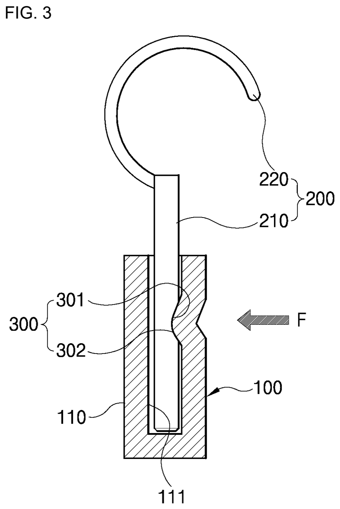

In another example, as illustrated in FIG. 3, the hollow 111 may be formed in a structure in which the hollow 111 is open only on one side in the longitudinal direction of the pipe body 110.

The coupler portion 200 may be arranged to come into contact with the incus 2.

One side of the coupler portion 200 may be arranged to come into contact with the incus 2, and the other side of the coupler portion 200 may be arranged in an inserted state inside the head portion 100.

In this way, a total length of the replacement prosthesis for the auditory ossicle may be set according to a degree to which the coupler portion 200 is inserted into the head portion 100.

For example, the coupler portion 200 may have a hook shape.

The coupler portion 200 may include a connector shaft 210 and a connector 220.

One end of the connector shaft 210 may be connected to the connector 220.

The connector 220 is provided at one end of the connector shaft 210 and comes into contact with the incus 2.

For example, the connector 220 may have a ring shape having an opening on one side, and accordingly, the connector 220 may be stably hooked on the incus 2.

Here, the head portion 100 and the coupler portion 200 may fix a length-adjusted position therebetween through a position fixing portion 300.

For example, the position fixing portion 300 may be a caulking that pressurizes a connection portion between the head portion 100 and the coupler portion 200 but is not limited thereto, and an adhesive may be used therefor.

A structure in which the position fixing portion 300 fixes the length-adjusted position between the head portion 100 and the coupler portion 200 through caulking may be composed of a catch protrusion 301 formed by pressurization on the inner circumferential surface of the pipe body 110 of the head portion 100, and a catch groove 302 formed in the coupler portion 200 to correspond to the catch protrusion 301.

Here, when the position fixing portion 300 is formed by a caulking that pressurizes the connection portion between the head portion 100 and the coupler portion 200, a shape of an outer surface is deformed due to pressurization of the head portion 100, and the deformation of the external shape provides a catch structure when performing a connection work on the stapes, and accordingly, more stable fixation may be provided.

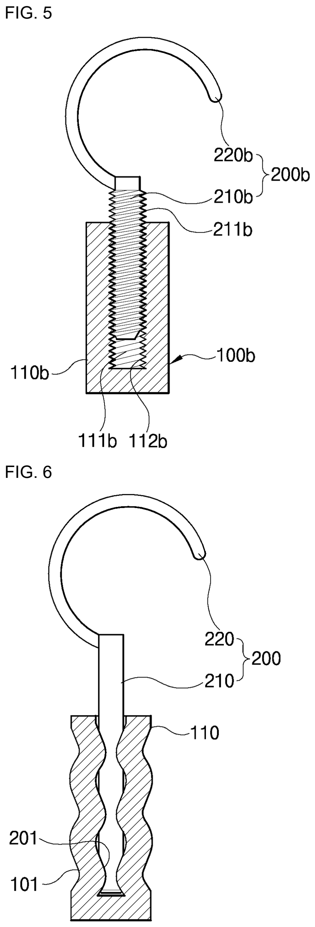

In addition, as illustrated in FIG. 6, cutting grooves 101 and 201 may be provided respectively on an outer surface of the head portion 100 and a surface of the connector shaft 210 of the coupler portion 200 to enable length adjustment through cutting.

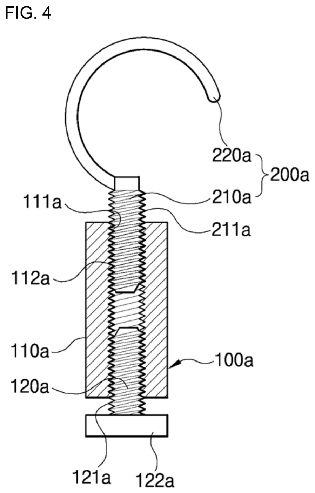

FIGS. 4 and 5 illustrate replacement prosthesis for an auditory ossicle, according to other embodiments, and the replacement prosthesis may include a head portion 100a and a coupler portion 200a.

Here, the head portion 100a may include a pipe body 110a and an adjustment pin 120a.

The pipe body 110a may have a hollow 111a, and a screw portion 112a may be provided on an inner surface of the pipe body 110a.

The adjustment pin 120a may be partially inserted into one side of the hollow 111a of the pipe body 110a.

A screw portion 121a is provided on an outer surface of the adjustment pin 120a and is fastened with the screw portion 112a in the hollow 111a of the pipe body 110a.

Here, the degree of insertion into the hollow 111a according to a rotation direction is adjusted when the adjustment pin 120a is inserted into one side of the hollow 111a, and the coupler portion 200a serves to limit a maximum depth that may be inserted into the other side of the hollow 111a of the head portion 100a.

In addition, one end of the adjustment pin 120a may be provided with a stopper end 122a that, when the adjustment pin is inserted into the hollow 111a of the pipe body 110a at a certain depth, catches an end portion of the pipe body 110a and prevents the adjustment pin 120a from being inserted further into the hollow 111a.

The coupler portion 200a may be arranged to come into contact with the incus 2.

One end of the coupler portion 200a may be arranged to come into contact with the incus 2, and the other end of the coupler portion 200a may be arranged to be inserted into the head portion 100a.

In this way, a total length of the replacement prosthesis for the auditory ossicle may be set according to the degree to which the coupler portion 200a is inserted into the head portion 100a.

For example, the coupler portion 200a may have a hook shape.

The coupler portion 200a may include a connector shaft 210a and a connector 220a.

One end of the connector shaft 210a may be connected to the connector 220a.

The connector shaft 210a may have the screw portion 211a that is fastened with the screw portion 112a formed in the hollow 111a of the head portion 100a.

In this way, because the connector shaft 210a is fastened with the screw portions 111a and 211a through the hollow 111a of the head portion 100a, a total length of the replacement prosthesis for the auditory ossicle may be adjusted according to the degree of insertion into the hollow 111a according to a rotation direction of the connector shaft 210a.

The connector 220a is provided at one end of the connector shaft 210a and comes into contact with the incus 2.

For example, the connector 220a may have a ring shape with an opening provided on one side, and accordingly, the connector 220a may be stably hooked on the incus 2.

Here, the head portion 100a and the coupler portion 200a may be stably fixed to each other through the position fixing portion 300 at the position adjusted in length through the screw portions 111a and 211a.

FIG. 5 is a replacement prosthesis for an auditory ossicle, according to another embodiment, and the replacement prosthesis may include a head portion 100b and a coupler portion 200b.

Here, the head portion 100b may include a head body 110b in which an insertion groove 111b is formed.

Here, a screw portion 112b may be provided on an inner surface of the head body 110b at which the insertion groove 111b is located.

The coupler portion 200b may be arranged to come into contact with the incus 2.

One side of the coupler portion 200b may be arranged to come into contact with the incus 2, and the other side of the coupler portion 200b may be arranged in a state of being inserted in the head portion 100b.

In this way, a total length of the replacement prosthesis for the auditory ossicle may be set according to the degree to which the coupler portion 200b is inserted into the head portion 100b.

For example, the coupler portion 200b may have a hook shape.

The coupler portion 200b may include a connector shaft 210b and a connector 220b.

One end of the connector shaft 210b may be connected to the connector 220b.

The connector shaft 210b may have a screw portion 211b that is fastened with the screw portion 112b formed in the insertion groove 111b of the head portion 100b.

In this way, because the connector shaft 210b is fastened with the screw portions 112b and 211b through the insertion groove 111b of the head portion 100b, a total length of the replacement prosthesis for the auditory ossicle may be adjusted according to the degree of insertion into the insertion groove 111b according to a rotation direction of the connector shaft 210b.

The connector 220b is provided at one end of the connector shaft 210b and comes into contact with the incus 2.

For example, the connector 220b has a ring shape with an opening provided on one side, and accordingly, the connector 220b may be stably hooked on the incus 2.

Here, the head portion 100b and the coupler portion 200b may be stably fixed to a position, of which length is adjusted through the screw portions 112b and 211b, through the position fixing portion 300 described above.

In this way, according to the replacement prosthesis for an auditory ossicle of the present embodiment, when the head portion 100 is arranged to come into contact with the stapes 1 instead of the auditory ossicle of a replacement part and the coupler portion 200 is arranged to come into contact with the incus 2, the head portion 100 and the coupler portion 200 are connected to each other such that a length may be adjusted, and accordingly, the replacement prosthesis may perform easy and stable treatment while being stably adjusted to a length of a patient's auditory ossicle to be replaced.

The present disclosure is described with reference to the embodiments illustrated in the drawings, but the embodiments are merely examples, and those skilled in the art will understand that various modifications and equivalent other embodiments may be derived therefrom. Therefore, the true technical protection scope of the present disclosure should be determined by the technical idea of the appended claims.

### Reference Signs List

100,100a,100b: head portion
200,200a,200b: coupler portion
210,210a,210b: connector shaft
220,220a,220b: connector

## Claims

1. A replacement prosthesis for an auditory ossicle, which connects an incus to a stapes, the replacement prosthesis comprising:
a head portion having one side arranged to come into contact with the stapes; and
a coupler portion connected to the head portion and having one side arranged to come into contact with the incus,
wherein the head portion is connected to the coupler portion such that a connection length is adjustable.

2. The replacement prosthesis of claim 1, further comprising:
a position fixing portion configured to fix a length-adjusted position between the head portion and the coupler portion.

3. The replacement prosthesis of claim 1, wherein
the position fixing portion is a caulking that pressurizes a connection portion between the head portion and the coupler portion.

4. The replacement prosthesis of claim 1, wherein
the head portion includes a pipe body having a hollow, and
another side of the coupler portion is inserted into one side of the hollow.

5. The replacement prosthesis of claim 4, wherein
the hollow is open on both sides in a longitudinal direction of the pipe body.

6. The replacement prosthesis of claim 4, wherein
the hollow is open on one side in a longitudinal direction of the pipe body.

7. The replacement prosthesis of claim 4, wherein
an inner surface of the pipe body and the coupler portion further include respectively screw portions that fasten the inner surface with the coupler portion.

8. The replacement prosthesis of claim 7, further comprising:
an adjustment pin that is fastened with another side of the hollow by a screw portion of the adjustment pin.

9. The replacement prosthesis of claim 1, wherein
the head portion includes a head body in which an insertion groove is formed, and
another side of the coupler portion is connected to the insertion groove by a screw portion of the coupler portion.

10. The replacement prosthesis of claim 1, wherein
the coupler portion includes a connector shaft connected to the head portion, and a connector provided at one end of the connector shaft to come into contact with the incus.

11. The replacement prosthesis of claim 9, wherein
the coupler portion has a hook shape.

## Amended claims

### Amended claims in accordance with Rule 137(2) EPC.

1. A replacement prosthesis (10) for an auditory ossicle, which connects an incus (2) to a stapes (1), the replacement prosthesis comprising:
a head portion (100) having one side arranged to come into contact with the stapes (1); and
a coupler portion (200) connected to the head portion (100) and having one side arranged to come into contact with the incus (2),
wherein the head portion (100) is connected to the coupler portion (200) such that a connection length is adjustable,
wherein the head portion (100) includes:
- a pipe body (110) having a hollow (111) open on both sides in a longitudinal direction of the pipe body (110),
wherein another side of the coupler portion (200) is inserted into one side of the hollow (111),
wherein an inner surface of the pipe body (110) and the coupler portion (200) further include respectively screw portions (112a, 211a) that fasten the inner surface with the coupler portion (200); and
- an adjustment pin (120a) that is fastened with another side of the hollow (111) by a screw portion (121a) of the adjustment pin (120a).

2. The replacement prosthesis (10) of claim 1, further comprising:
a position fixing portion (300) configured to fix a length-adjusted position between the head portion (100) and the coupler portion (200).

3. The replacement prosthesis (10) of claim 2, wherein
the position fixing portion (300) is a caulking that pressurizes a connection portion between the head portion (100) and the coupler portion (200).

4. The replacement prosthesis (10) of claim 1, wherein
the coupler portion (200) includes a connector shaft (210) connected to the head portion (100), and a connector (220) provided at one end of the connector shaft (210) to come into contact with the incus (2).

5. The replacement prosthesis (10) of claim 1, wherein
the coupler portion (200, 200a) has a hook shape.
